# EUROPEAN PATENT APPLICATION

(11) **EP 1 978 096 A1**
(43) Date of publication of application: **08.10.2008**
(21) Application number: 06835068.5
(22) Date of filing: 21.12.2006
(51) Int. Cl.: C12N 15/09, A61K 31/7125, A61K 48/00, A61P 3/04, A61P 9/00, A61P 9/10, A61P 11/00, A61P 11/06, A61P 13/12, A61P 17/00, A61P 17/02, A61P 17/06, A61P 19/02, A61P 19/08, A61P 29/00, A61P 35/00, A61P 35/04, A61P 37/00, A61P 37/08

(54) **NOVEL OLIGONUCLEOTIDE AND NF-KAPPA B DECOY COMPRISING THE SAME**

(30) Priority: 22.12.2005 JP 2005370188
(71) Applicant: Genomidea Inc, Ibaraki-shi, Osaka 567-0085 (JP); AnGes MG, Inc., Ibaraki-shi Osaka 567-0085 (JP)
(72) Inventor: NAKAJIMA, Toshihiro, Ibaraki-shi, Osaka 567-0085 (JP); TEMMA, Akiko, Ibaraki-shi, Osaka 567-0085 (JP); SUZUKI, Naho, Ibaraki-shi, Osaka 567-0085 (JP)
(74) Representative: Gille Hrabal Struck Neidlein Prop Roos
(86) International application number: PCT/JP2006/325502
(87) International publication number: WO 2007/072909

(57) **Abstract**

A novel oligonucleotide useful as an NF-κB decoy having a higher binding ability to NF-κB than known NF-κB decoy as well as the medical uses thereof, is disclosed. The oligonucleotide of the invention has a base sequence having a consensus sequence and specific 5'-flanking and 3'-flanking sequences ligated to both ends of the consensus sequence, respectively. The NF-κB decoy is constituted by an oligonucleotide which is the above-described oligonucleotide of the invention and which is substantially double-stranded wherein the strands are complementary to each other.

## Description

### Technical Field

The present invention relates to a novel oligonucleotide and to an NF-κB decoy comprising the same. The NF-κB decoy according to the present invention is useful for the prophylaxis, amelioration and/or therapy of ischemic diseases, allergic diseases, autoimmune diseases, and metastasis/infiltration of cancers.

### Background Art

NF-κB (nuclear factor kappa B) is a collective name of a family of transcription factors, which have a role in regulating expression of the genes involved in immunoreactions. When NF-κB binds to the binding site in the genomic gene, the genes involved in immunoreactions are overexpressed. Therefore, NF-κB is known to be involved in various diseases such as allergic diseases such as atopic dermatitis and rheumatoid arthritis, and autoimmune diseases, which are caused by immunoreactions, as well as in ischemic diseases such as myocardial infarction and arteriosclerosis.

On the other hand, it is known to administer a decoy for a transcription factor to decrease the activity of the transcription factor, thereby performing therapy or prophylaxis of diseases caused by the transcription factor. The term decoy is an English word which means "decoy", and one having the structure similar to that which a substance binds to or acts on is called a decoy. As the decoys for transcription factors which bind to a binding region in a genomic gene, double-stranded oligonucleotides having the same base sequence as the binding region are mainly used (Patent Literatures 1 to 3). In the presence of a decoy constituted by such an oligonucleotide, a part of the transcription factor molecules binds to the oligonucleotide decoy rather than binding to the binding region in the genomic gene to which the transcription factor should normally bind. As a result, the number of transcription factor molecules bound to the binding site in the genomic gene to which they should normally bind is decreased, so that the activity of the transcription factor is decreased accordingly. In this case, since the oligonucleotide functions as an imitation (decoy) of the real binding site in the genomic gene and binds to the transcription factor, it is called a decoy. Various oligonucleotide decoys for NF-κB are known, and various pharmacological activities thereof are also known (Patent Literatures 4 to 12).

It is well-known that it is also an important key for making the above-described mechanism effectively work that the decoy oligonucleotide delivered into the cells can exist stably in the cells for a long time. Since oligonucleotides become degraded by nucleases in the cells, it is difficult to make the oligonucleotides stably exist in the cells and in the nuclei. To overcome this difficult problem, methods in which various modifications are given to the bases in the oligonucleotides have been tried (for example, Non-patent Literature 1 and Patent Literature 13). Among these modifications, the most frequently used modification is the modification by phosphorothioation (PS). Since phosphorothioated oligonucleotides are highly resistant to nucleases, they draw attention as oligonucleotides for therapies (for example, Non-patent Literature 2). Phosphorothioation is to replace one of the two non-crosslinking oxygen atoms bound to the phosphorus atom constituting the phosphodiester linkage between adjacent nucleotides with a sulfur atom.

However, while phosphorothioated oligonucleotides have much higher resistance to nucleases than the natural phosphodiester oligonucleotides, the disadvantages that the binding capacity to the target molecule is decreased when compared with the phosphodiester oligonucleotides, and it is observed in many cases that the specificity to the target molecule is decreased (Non-patent Literature 1 and Non-patent Literature 3). Further, since phosphorothioate group is toxic, in many cases, phosphorothioated oligonucleotides have higher cytotoxicity than phosphodiester oligonucleotides (Non-patent Literature 4). This is also a disadvantage of the phosphorothioated oligonucleotides when used as therapeutic agents.

Patent Literature 1: Japanese PCT Patent Application Re-laid-open No. 96/035430
Patent Literature 2: JP 3392143 B
Patent Literature 3: WO95/11687
Patent Literature 4: JP 2005-160464 A
Patent Literature 5: WO96/35430
Patent Literature 6: WO02/066070
Patent Literature 7: WO03/043663
Patent Literature 8: WO03/082331
Patent Literature 9: WO03/099339
Patent Literature 10: WO04/026342
Patent Literature 11: WO05/004913
Patent Literature 12: WO05/004914
Patent Literature 13: Japanese Translated PCT Patent Application Laid-open No. 08-501928
Non-patent Literature 1: Milligan et al., J. Med. Chem. 1993, 36, 1923
Non-patent Literature 2: Marwick, C.,(1998) J. Am. Med. Assoc., 280, 871
Non-patent Literature 3: Stein & Cheng, Science 1993, 261, 1004
Non-patent Literature 4: Levin et al., Biochem. Biophys. Acta, 1999, 1489, 69
Non-patent Literature 5: Neish AS et al., J. Exp. Med. 1992, Vol. 176, 1583-1593.
Non-patent Literature 6: Leung K et al., Nature. 1988 Jun 23;333(6175):776-778.) Non-patent Literature 7: Marina A. et al., The Journal of Biological Chemistry, 1995, Vol.270, Number 6, pp. 2620-2627

### Disclosure of the Invention

### Problems to be Solved by the Invention

Although oligonucleotide decoys for NF-κB are known, it is desired, needless to say, to provide an oligonucleotide decoy having a higher binding capacity to NF-κB than the known oligonucleotide decoys. Accordingly, an object of the present invention is to provide a novel oligonucleotide useful as an NF-κB decoy, which oligonucleotide has a higher binding capacity to NF-κB than the known oligonucleotide decoys, as well as medical uses thereof.

Another object of the present invention is to provide an oligonucleotide decoy for a transcription factor, which decoy has a high binding capacity to the target transcription factor and which also has a resistance to nucleases.

### Means for Solving the Problems

In the prior art, much efforts have been directed to the improvement of the region to which NF-κB binds. The present inventors thought that the base sequences of the regions adjacent to the region to which NF-κB binds may play an important role in the capacity to bind to NF-κB. Thus, as will be concretely described in Examples below, the present inventors prepared as many as 100 types of oligonucleotides as the regions adjacent to the same binding region, and the capacities thereof to bind to NF-κB were tested to discover oligonucleotides having high capacities to bind to NF-κB, thereby completing the present invention.

Further, the present inventors discovered that by subjecting only the core sequence of an oligonucleotide decoy to a nuclease-resistant modification, the binding capacity of the decoy to the transcription factor is largely increased when compared to the cases where the entire sequence is completely subjected to the nuclease-resistant modification, thereby completing the second invention of the present application.

Accordingly, the present invention provides an oligonucleotide having a base sequence represented by the following Formula [I]:

A-X-B [I]

(wherein in Formula [I], X is a consensus sequence represented by gggatttccc or gggactttcc; A is a 5'-flanking sequence selected from the group consisting of cgc, ccc, gga, cgca, ccct and ggct; and B is a 3'-flanking sequence selected from the group consisting of age, acc, ggg, gcg, gcc and gcgg). The present invention also provides an NF-κB decoy constituted by the above-described oligonucleotide of the present invention, in which the oligonucleotide is substantially double-stranded wherein the strands constituting the double strands are complementary to each other. The present invention further provides a pharmaceutical comprising the oligonucleotide of the present invention as an active ingredient, in which the oligonucleotide is substantially double-stranded wherein the strands constituting the double strands are complementary to each other. The present invention still further provides a method for inhibiting NF-κB, in which the method comprises having the oligonucleotide of the present invention interact with NF-κB, the oligonucleotide being substantially double-stranded wherein the strands constituting the double strands are complementary to each other. The present invention still further provides use of the oligonucleotide of the present invention for the production of an inhibitor for inhibiting NF-κB, in which the oligonucleotide is substantially double-stranded wherein the strands are complementary to each other. The present invention still further provides a method for prophylaxis, amelioration and/or therapy of a disease which is cured or ameliorated by inhibition of NF-κB, wherein the method comprises administering an effective amount of the oligonucleotide of the present invention, which is substantially double-stranded wherein the strands are complementary to each other. The present invention still further provides use of the oligonucleotide of the present invention, which is substantially double-stranded wherein the strands are complementary to each other for the production of a pharmaceutical for a disease which is cured or ameliorated by inhibition of NF-κB.

Further, the present invention provides an oligonucleotide decoy for a transcription factor, constituted by an oligonucleotide which is substantially double-stranded wherein the strands are complementary to each other, the oligonucleotide comprising a core sequence and a flanking sequence(s) ligated to one or both ends of the core sequence, characterized in that the bonds between only all of the nucleotides constituting the consensus sequence are modified by a nuclease-resistant modification, and the bonds between all of other nucleotides are not modified. The present invention also provides a method for inhibiting a transcription factor, wherein the method comprises making an effective amount of an oligonucleotide decoy for the transcription factor interact with the transcription factor, in which the oligonucleotide decoy is constituted by an oligonucleotide having a core sequence and a flanking sequence(s) ligated to one or both ends of the core sequence, characterized in that the bonds between only all of the nucleotides constituting the consensus sequence are modified by a nuclease-resistant modification, and the bonds between all of other nucleotides are not modified. The present invention further provides use of an oligonucleotide decoy for the production of an inhibitor of a transcription factor, wherein the oligonucleotide decoy being constituted by an oligonucleotide having a core sequence and a flanking sequence(s) ligated to one or both ends of the core sequence, characterized in that the bonds between only all of the nucleotides constituting the consensus sequence are modified by a nuclease-resistant modification, and the bonds between all of other nucleotides are not modified.

### Effects of the Invention

By the present invention, a novel oligonucleotide having a higher capacity to bind to NF-κB than the known decoy oligonucleotides was provided. Since the oligonucleotide of the present invention has a high capacity to bind to NF-κB, the oligonucleotide exhibits a better performance as a decoy for NF-κB than the known oligonucleotides, and can decrease the physiological activity of NF-κB to a lower level. Therefore, the various pharmaceuticals comprising the decoy of the present invention as an active ingredient exhibits superior pharmacological effects.

According to the second invention which is an oligonucleotide decoy in which the bonds between only all of the nucleotides constituting the consensus sequence are modified by a nuclease-resistant modification, as will be shown concretely in the Examples below, the binding capacity to the transcription factor is much higher than the fully phosphorothioated oligonucleotide having the same base sequence. On the other hand, since the core sequence constituting the central part of the oligonucleotide is resistant to nucleases by phosphorothioation, the resistance to nucleases is not decreased very much when compared with the fully phosphorothioated oligonucleotide. Therefore, it is believed that the partially phosphorothioated oligonucleotides exhibit superior performance as a decoy for transcription factors *in vivo*.

### Best Mode for Carrying out the Invention

As described above, the oligonucleotide of the present invention has the base sequence represented by the above-described Formula [I]. In the present invention, the term "has the base sequence" means that the bases are aligned in the order described. Thus, for example, "the oligonucleotide having the base sequence of cgcgggatttcccagc" means the oligonucleotide having a size of 16 bases having a base sequence of cgcgggatttcccagc. The oligonucleotide having the base sequence represented by Formula [I] includes the single-stranded oligonucleotide having the base sequence described, the oligonucleotide which is the complementary strand of the above-mentioned single-stranded oligonucleotide, double-stranded oligonucleotide in which the strands are complementary to each other, and partially double-stranded oligonucleotides in which a part of the above-mentioned single-stranded oligonucleotide is hybridized with a complementary strand thereof. In the present Description and the Claims, "double-stranded oligonucleotide wherein the strands are complementary to each other" means a complete double-stranded oligonucleotide in which the full length of the oligonucleotide is double-stranded, and constituted by strands complementary to each other. As described below, in cases where the oligonucleotide is used as a decoy for NF-κB (which may also be referred to as "NF-κB decoy" in the present Description and Claims), the oligonucleotide is preferably a double-stranded oligonucleotide wherein the strands are complementary to each other.

In Formula [I], the consensus sequence represented by X is gggatttccc (SEQ ID NO:1, Non-patent Literature 5) or gggactttcc (SEQ ID NO:2, Non-patent Literature 6), and the sequence of SEQ ID NO:1 is preferred. These consensus sequences have the base sequence of the binding region in the genomic genes to which the NF-κB family commonly binds.

Since the "A" in Formula [I] is a sequence flanking the 5'-end of the consensus sequence, it is called "5'-flanking sequence" in the present invention, and is selected from the group consisting of cgc, ccc, gga, cgca, ccct and ggct. Since the "B" in Formula [I] is a sequence flanking the 3'-end of the consensus sequence, it is called "3'-flanking sequence" in the present invention, and is selected from the group consisting of agc, acc, ggg, gcg, gcc and gcgg.

Preferred examples of the oligonucleotides represented by Formula [I] includes cgcgggatttcccagc (SEQ ID NO:3), cccgggatttcccacc (SEQ ID NO:4), ggagggatttcccggg (SEQ ID NO:5), cgcagggatttcccgcg (SEQ ID NO:6), ccctgggatttcccgcc (SEQ ID NO:7) and ggctgggatttcccgcgg (SEQ ID NO:8).

Although the oligonucleotides of the present invention are preferably DNAs basically, it is preferred to modify a bond(s) between at least two adjacent nucleotides by a nuclease-resistant modification to increase the resistance to nucleases. The term "nuclease-resistant modification" herein means a modification by which the DNA is more unlikely degraded by the nucleases than the natural DNAs. Such modifications *per se* are well-known. Examples of the nuclease-resistant modifications include phosphorothioation (which may be referred to as "phosphorothioation" in the present Description), phosphorodithioation, phosphoroamidation and the like. Among these, phosphorothioation is preferred. As described above, phosphorothioation means to replace one of the two non-crosslinking oxygen atoms bound to the phosphorus atom constituting the phosphoester bond between adjacent nucleotides with a sulfur atom. The methods *per* se to phosphorothioate the bond between arbitrary adjacent bases are well-known, and phosphorothioation may be carried out by, for example, the method described in Non-patent Literature 7. Phosphorothioated oligonucleotides are also commercially synthesized. In the oligonucleotides of the present invention, although the oligonucleotides in which all bonds between all nucleotides are phosphorothioated (which may also be referred to as "fully phosphorothioated oligonucleotide") are also preferred, the oligonucleotides are more preferred in which bonds between only all of the nucleotides constituting the consensus sequence are phosphorothioated, and the bonds between all of other nucleotides, that is, the bonds between the nucleotides constituting the 5'-flanking sequence, the bonds between the nucleotides constituting the 3'-flanking sequence, the bond between the nucleotide at the 5'-end of the consensus sequence and the nucleotide at the 3'-end of the 5'-flanking sequence, and the bond between the nucleotide at the 3'-end of the consensus sequence and the nucleotide at the 5'-end of the 3'-flanking sequence, are not phosphorothioated (such an oligonucleotide may also be hereinafter referred to as "partially phosphorothioated oligonucleotide" in the present Description). As will be concretely described in the Examples below, the partially phosphorothioated oligonucleotides have a capacity to bind to NF-κB which is 3 to 5 times higher than the fully phosphorothioated oligonucleotides having the same sequence. On the other hand, since the core sequence constituting the central part of the oligonucleotide is resistant to nucleases by phosphorothioation, the resistance to nucleases is not decreased very much when compared with the fully phosphorothioated oligonucleotide. Therefore, it is believed that the partially phosphorothioated oligonucleotides exhibit superior performance as a decoy for transcription factors *in vivo*. In case of a double-stranded oligonucleotide, although the above-described nuclease-resistant modification may be performed on only either one of the strands, it is preferred to perform the nuclease-resistant modification on both strands.

The oligonucleotides of the present invention may be synthesized with a commercially available nucleic acid synthesizer. The oligonucleotides may be prepared in a large amount by a nucleic acid-modification method such as PCR.

The oligonucleotides of the present invention, which are substantially double-stranded wherein the strands are complementary to each other, have a use as an NF-κB decoy. Thus, the present invention also provides an NF-κB decoy constituted by the oligonucleotide of the present invention, which oligonucleotide is substantially double-stranded wherein the strands are complementary to each other. The term "NF-κB" means a homo or hetero dimer of a protein included in the NF-κB/Rel family members. The term "NF-κB" family" means proteins in NF-κB/Rel family members, such as, for example, P50, P52, P65(Rel-A), c-Rel and Rel-B. The term "homo or hetero dimer" includes any combination of the proteins included in the NF-κB family member. The term "substantially double-stranded" herein means that the oligonucleotide is completely double-stranded, or one or two nucleotides at an end of at least one strand are single-stranded. Although substantially double-stranded oligonucleotides can be used as an NF-κB decoy, those completely double-stranded are preferred. Single-stranded oligonucleotides have uses as a template in the nucleic acid-amplification methods, and as a ligand used for purifying the oligonucleotides of the present invention by an affinity chromatography. Partially double-stranded oligonucleotides have a use as the starting material when a substantially double-stranded oligonucleotide is generated, or when single-stranded oligonucleotides are formed by denaturation.

As described above, various NF-κB decoys are known, and various medical uses thereof are also known. Thus, the NF-κB decoy of the present invention has the same medical uses as the known NF-κB decoys. More particularly, the NF-κB decoy of the present invention has medical uses as an agent for prophylaxis, amelioration and/or therapy of ischemic diseases, allergic diseases, inflammatory diseases, autoimmune diseases, metastasis/infiltration of cancers, or cachexy; as an agent for prophylaxis, amelioration and/or therapy of vascular restenosis, acute coronary syndrome, brain ischemia, myocardial infarction, reperfusion hindrance of ischemic diseases, atopic dermatitis, psoriasis vulgaris, contact dermatitis, keloid, decubital ulcer, ulcerative colitis, Crohn's disease, nephropathy, glomerulosclerosis, albuminuria, nephritis, renal failure, rheumatoid arthritis, osteoarthritis, degenerative intervertebral disc, asthma, chronic obstructive pulmonary disease or cystic fibrosis; and as an agent for prophylaxis, amelioration and/or therapy of vascular restenosis which occurs after percutaneous transluminal coronary angioplasty, percutaneous transluminal angioplasty, bypass surgery, organ transplantation or surgery of an organ. The "vascular restenosis" mentioned above includes those caused by using an artificial blood vessel, catheter or stent, or by vein grafting; and those caused by a surgical treatment for arteriosclerosis obliterans, aneurysm, aorta dissection, acute coronary syndrome, brain ischemia, Marfan syndrome or plaque rupture.

When the oligonucleotide is applied for these medical uses, the administration route of the oligonucleotide is not restricted, and parenteral administration such as intravenous administration, intramuscular administration, subcutaneous administration, percutaneous administration or direct administration to the target organ or tissue is preferred. The dose of administration may be appropriately selected depending on the disease to be treated, the conditions of the patient, the administration route and so on, and the dose per adult per day is usually 0.1 to 10000 nmol, preferably 1 to 1000 nmol, more preferably 10 to 100 nmol. Formulation may be attained by conventional methods. For example, in case of an injection solution, the injection solution may be in the form of a solution formulated by dissolving the oligonucleotide of the present invention in physiological saline. The formulation may appropriately contain other additive(s) conventionally used in the field of formulation, such as preservatives, buffering agents, solubilizers, emulsifiers, diluents, isotonic agents and the like. The formulation may also contain other pharmaceutical component(s).

As described above and as will be described concretely in the Examples below, the above-described partially phosphorothioated oligonucleotide decoys have a higher binding capacity to the transcription factor than the fully phosphorothioated oligonucleotide decoys, and on the other hand, since the core sequence constituting the central part of the oligonucleotide is resistant to nucleases by phosphorothioation, the resistance to nucleases is not decreased very much when compared with the fully phosphorothioated oligonucleotides. Therefore, it is believed that the partially phosphorothioated oligonucleotides exhibit superior performance as a decoy for transcription factors *in vivo*. Thus, the present invention also provides an oligonucleotide decoy for a transcription factor, constituted by an oligonucleotide which is substantially double-stranded wherein the strands are complementary to each other, the oligonucleotide comprising a core sequence and a flanking sequence(s) ligated to one or both ends of the core sequence, characterized in that the bonds between only all of the nucleotides constituting the consensus sequence are modified by a nuclease-resistant modification, and the bonds between all of other nucleotides are not modified. The term "core sequence" herein means the region to which the transcription factor binds, and in case of an NF-κB, it is the above-described consensus sequence. The meaning of the term "substantially double-stranded" is the same as described above, and fully double-stranded ones are preferred. Although the above-described nuclease-resistant modification may be performed on only either one of the strands, it is preferred to perform the nuclease-resistant modification on both strands. Examples of the transcription factors include, but not limited to, STAT-1, STAT-3, STAT-6, Ets, AP-1, E2F and the like, in addition to those belonging to the NF-κB family.

The present invention will now be described more concretely by way of Examples thereof. It should be noted, however, the present invention is not restricted to the Examples below.

### Examples

### 1. Preparation of Oligonucleotides

One hundred types of oligodeoxynucleotides (hereinafter also referred to as "ODN") were chemically synthesized, each of which comprises flanking sequences ligated to the both ends of the sequence shown in SEQ ID NO:1 which is a known consensus sequence of NF-κB. Two strands complementary to each other were chemically synthesized respectively, and the synthesized strands were hybridized to form a completely double-stranded ODN. In each ODN, the bonds between all of the nucleotides of both strands were phosphorothioated (hereinafter also referred to as "SODN"). The ODN number, base sequence, SEQ ID NO, size and melting temperature (Tm) of each thereof are shown in Tables 1-1 to 1-3. Among the 100 types of oligonucleotides shown in Tables 1-1 to 1-3, the oligonucleotides of the present invention represented by Formula [I] are SODN7 (SEQ ID NO:3), SODN8 (SEQ ID NO:4), SODN9 (SEQ ID NO:5), SODN16 (SEQ ID NO:6), SODN17 (SEQ ID NO:7) and SODN30 (SEQ ID NO:8), that is, totally 6 types of oligonucleotides.

**Table 1-1**

| SODN No. | SEQ ID NO | Number of Bases | Tm (°C) | Sequence (5' → 3') |
|---|---|---|---|---|
| 1 | 9 | 16 | 50 | cttgggatttcccgtc |
| 2 | 10 | 16 | 50 | gtagggatttcccgtg |
| 3 | 11 | 16 | 50 | cgtgggatttcccttc |
| 4 | 12 | 16 | 50 | ctcgggatttcccatc |
| 5 | 13 | 16 | 50 | cttgggatttccctcc |
| 6 | 14 | 16 | 54 | cgagggatttcccggc |
| 7 | 3 | 16 | 54 | cgcgggatttcccagc |
| 8 | 4 | 16 | 54 | cccgggatttcccacc |
| 9 | 5 | 16 | 54 | ggagggatttcccggg |
| 10 | 15 | 16 | 54 | cctgggatttcccgcc |
| 11 | 16 | 17 | 54 | gttcgggatttccctgc |
| 12 | 17 | 17 | 54 | cctcgggatttcccatc |
| 13 | 18 | 17 | 54 | cacagggatttcccgtc |
| 14 | 19 | 17 | 54 | ccgtgggatttcccttc |
| 15 | 20 | 17 | 54 | caccgggatttcccaac |
| 16 | 6 | 17 | 58 | cgcagggatttcccgcg |
| 17 | 7 | 17 | 58 | ccctgggatttcccgcc |
| 18 | 21 | 17 | 58 | cggcgggatttccctgg |
| 19 | 22 | 17 | 58 | cggagggatttcccggg |
| 20 | 23 | 17 | 58 | ggccgggatttccctcg |
| 21 | 24 | 18 | 54 | ctgagggatttcccattc |
| 22 | 25 | 18 | 54 | ctttgggatttccctgtc |
| 23 | 26 | 18 | 54 | catagggatttcccatcc |
| 24 | 27 | 18 | 54 | gctagggatttcccatag |
| 25 | 28 | 18 | 54 | gtctgggatttccctttg |
| 26 | 29 | 18 | 62 | gggtgggatttcccgggg |
| 27 | 30 | 18 | 62 | cgcagggatttcccgcgc |
| 28 | 31 | 18 | 62 | cggtgggatttcccgggc |
| 29 | 32 | 18 | 62 | cgccgggatttcccacgc |
| 30 | 8 | 18 | 62 | ggctgggatttcccgcgg |
| 31 | 33 | 19 | 58 | cgcgggatttcccaatatc |
| 32 | 34 | 19 | 58 | ctagggatttcccaccttc |
| 33 | 35 | 19 | 58 | cgcgggatttccctattag |
| 34 | 36 | 19 | 58 | gtagggatttcccgtgaac |
| 35 | 37 | 19 | 58 | cttgggatttcccgcttag |

**Table 1-2**

| SODN No. | SEQ ID NO | Number of Bases | Tm (°C) | Sequence (5' → 3') |
|---|---|---|---|---|
| 36 | 38 | 19 | 62 | ctcgggatttcccagctcg |
| 37 | 39 | 19 | 62 | ctagggatttcccgctggc |
| 38 | 40 | 19 | 62 | gaagggatttcccggtccc |
| 39 | 41 | 19 | 62 | cccgggatttccctaaccc |
| 40 | 42 | 19 | 62 | cacgggatttcccagcgac |
| 41 | 43 | 19 | 58 | gataccgggatttcccatg |
| 42 | 44 | 19 | 58 | catcgtgggatttcccttc |
| 43 | 45 | 19 | 58 | gaatgagggatttcccgtg |
| 44 | 46 | 19 | 58 | ggaacagggatttcccaag |
| 45 | 47 | 19 | 58 | gtcttagggatttcccacc |
| 46 | 48 | 19 | 62 | ggtcacgggatttccctgc |
| 47 | 49 | 19 | 62 | ctgtgcgggatttccctgc |
| 48 | 50 | 19 | 62 | cacctcgggatttccctcc |
| 49 | 51 | 19 | 62 | ccacgagggatttcccagc |
| 50 | 52 | 19 | 62 | gcaaccgggatttcccacc |
| 51 | 53 | 20 | 58 | gcagggatttcccattaaac |
| 52 | 54 | 20 | 58 | catgggatttccctcttaac |
| 53 | 55 | 20 | 58 | gtagggatttcccagttttc |
| 54 | 56 | 20 | 58 | gtagggatttcccagtatac |
| 55 | 57 | 20 | 58 | cttgggatttccctttcttc |
| 56 | 58 | 20 | 62 | ctcgggatttcccattcctc |
| 57 | 59 | 20 | 62 | gtcgggatttccctggtttg |
| 58 | 60 | 20 | 62 | cgtgggatttcccggatatc |
| 59 | 61 | 20 | 62 | cttgggatttcccggttagc |
| 60 | 62 | 20 | 62 | gttgggatttccctctgagg |
| 61 | 63 | 20 | 58 | catagggatttcccatcttg |
| 62 | 64 | 20 | 58 | ctttgggatttccctgtttg |
| 63 | 65 | 20 | 58 | ctctgggatttccctttatc |
| 64 | 66 | 20 | 58 | cttagggatttcccatgatc |
| 65 | 67 | 20 | 58 | gtttgggatttcccttgttc |
| 66 | 68 | 20 | 62 | catcgggatttcccaccttc |
| 67 | 69 | 20 | 62 | cttcgggatttcccaccttg |
| 68 | 70 | 20 | 62 | gtgagggatttcccgatgtc |
| 69 | 71 | 20 | 62 | gtaagggatttcccggctag |
| 70 | 72 | 20 | 62 | gctagggatttcccagtagc |

**Table 1-3**

| SODN No. | SEQ ID NO | Number of Bases | Tm (°C) | Sequence (5' → 3') |
|---|---|---|---|---|
| 71 | 73 | 20 | 58 | gatatgggatttcccactag |
| 72 | 74 | 20 | 58 | ctttcgggatttcccatttg |
| 73 | 75 | 20 | 58 | gttttgggatttcccttctc |
| 74 | 76 | 20 | 58 | gatacgggatttcccaatac |
| 75 | 77 | 20 | 58 | gattcgggatttcccttttg |
| 76 | 78 | 20 | 62 | ggtacgggatttcccactac |
| 77 | 79 | 20 | 62 | gggtcgggatttcccatatg |
| 78 | 80 | 20 | 62 | gttacgggatttccctctcc |
| 79 | 81 | 20 | 62 | ccctcgggatttcccaaatc |
| 80 | 82 | 20 | 62 | gtgatgggatttcccgttgg |
| 81 | 83 | 20 | 58 | gttcttgggatttccctttc |
| 82 | 84 | 20 | 58 | gtatatgggatttccctagg |
| 83 | 85 | 20 | 58 | caagtagggatttcccatac |
| 84 | 86 | 20 | 58 | gatattgggatttcccttcc |
| 85 | 87 | 20 | 58 | gtttttgggatttccctgtc |
| 86 | 88 | 20 | 62 | cgaattgggatttccctccg |
| 87 | 89 | 20 | 62 | gtttatgggatttcccgcgg |
| 88 | 90 | 20 | 62 | caatcagggatttcccgtcc |
| 89 | 91 | 20 | 62 | cgtttcgggatttccctctg |
| 90 | 92 | 20 | 62 | ggttgtgggatttcccgatg |
| 91 | 93 | 20 | 58 | gtaaaatgggatttcccgag |
| 92 | 94 | 20 | 58 | ctgaatagggatttcccatc |
| 93 | 95 | 20 | 58 | gaattctgggatttccctac |
| 94 | 96 | 20 | 58 | ctttttagggatttcccagc |
| 95 | 97 | 20 | 58 | gtaattagggatttcccagg |
| 96 | 98 | 20 | 62 | gctgtttgggatttcccgtc |
| 97 | 99 | 20 | 62 | gcaatacgggatttcccagg |
| 98 | 100 | 20 | 62 | caagtatgggatttcccggc |
| 99 | 101 | 20 | 62 | gagtcgagggatttcccatc |
| 100 | 102 | 20 | 62 | cttgtcagggatttcccacg |

### 2. Measurement of Binding Capacity to NF-κB (Primary Screening)

The binding capacity of each SODN to NF-κB was evaluated by measuring the remaining free NF-κB after reacting each SODN with NF-κB (p65), using a commercially available kit (TransAM Kit (NF- κB, p65, ACTIVE MOTIF) for measuring NF-κB, and using NF-κB molecules in the Jurkat, TPA and CI-Stimulated, Nuclear Extract (nuclear extract of Jurkat cells stimulated with a phorbol ester (TPA) and a calcium ionophore (CI)). The measurement was performed in accordance with the instructions attached to the kit. The kit was made for quantifying the NF-κB bound to the solid phase by ELISA after adding an NF-κB solution to the wells in which the NF-κB (p65 protein) consensus binding sequence was immobilized and after washing the resultant. By this measurement method, the higher the binding capacity of the oligonucleotide to the NF-κB, the smaller amount of the NF-κB quantified by the ELISA.

More concretely, the above-described measurement was carried out as follows concretely: Each SODN solution was serially diluted with Complete Binding Buffer contained in the kit to prepare test samples (common ratio: 3-fold, 4 times, n=3). To each well, 30 µL of the test sample was added. To the wells of the control and blank, Complete Binding Buffer was added. To each well, 20 µL of the Jurkat, TPA and CI-Stimulated, Nuclear Extract contained in the kit, after dilution with Complete Lysis Buffer to a concentration of 125 µg/mL, was added. To the wells of the blank, Complete Lysis Buffer was added. After incubation for 1 hour with shaking, each well was washed with 1X Wash Buffer contained in the kit, and an anti-NF-κB (p65 protein) antibody was added, followed by incubation for 1 hour. Each well was washed with 1X Wash Buffer, and the Developing Solution contained in the kit was added. After allowing coloration for 10 minutes, the Stop Solution was added to stop the reaction, and the absorbances at 450 nm and 630 nm were measured.

The absorbance at 630 nm was subtracted from the absorbance at 450 nm and the mean value of the blanks was further subtracted from the resultant, and the percentage of the mean value of each concentration based on the mean value of the controls was calculated. The concentration at which the calculated value was 50% (i.e., at which the binding was inhibited by 50%) was calculated from the regression lines between two points interposing 50% (an analysis software Graph Pad PRISM 4, GraphPad SOFTWARE was used). As the control, fully phosphorothioated ccttgaagggatttccctcc (SEQ ID NO:103) which is a known NF-κB decoy oligonucleotide was used. The results of the 10 types of SODN (SODNs 6, 7, 8, 9, 16, 17, 27, 30, 36 and 91) which showed high activities, as well as the results of 2 types of SODN (SODNs 82 and 83) which showed low activities, are shown in Table 2. The reason why the IC₅₀ values of the control decoy varied is that experiments were carried out dividing the 100 types of SODN into 18 groups, and the control value was measured in each run.

**Table 2**

| SODN No. | SEQ ID NO | Number of Bases | IC₅₀ (nM) | Percent (%) to Control Decoy | IC₅₀ (nM) of Control Decoy |
|---|---|---|---|---|---|
| 6 | 14 | 16 | 1.31 | 55.0 | 2.39 |
| 7 | 3 | 16 | 1.61 | 33.2 | 4.86 |
| 8 | 4 | 16 | 2.01 | 41.3 | 4.86 |
| 9 | 5 | 16 | 2.01 | 41.3 | 4.86 |
| 16 | 6 | 17 | 1.37 | 39.2 | 3.50 |
| 17 | 7 | 17 | 1.31 | 37.4 | 3.50 |
| 27 | 30 | 18 | 2.23 | 48.2 | 4.64 |
| 30 | 8 | 18 | 1.33 | 28.7 | 4.64 |
| 36 | 38 | 19 | 2.10 | 53.8 | 3.91 |
| 82 | 84 | 20 | >5 | - | 2.70 |
| 83 | 85 | 20 | >5 | - | 2.70 |
| 91 | 93 | 20 | 3.06 | 43.4 | 7.06 |

### 3. Measurement of Binding Capacity to NF-κB (Secondary Screening)

The 10 types of SODN (SODNs 6, 7, 8, 9, 16, 17, 27, 30, 36 and 91) which showed high activities in the primary screening and the 2 types of SODN (SODNs 82 and 83) which showed low activities in the primary screening were tested for the binding inhibition activities to NF-κB (p65 protein) by the same method as in the primary screening, under the conditions of common ratio of 3-fold, 6 times, n=3, and the results were compared. As a control for comparison, fully phosphorothioated ccttgaagggatttccctcc (SEQ ID NO:103) was used. The results are shown in Tables 3-1 and 3-2.

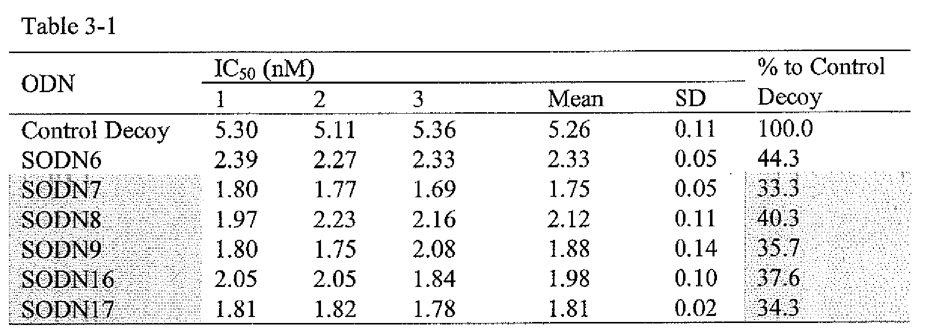

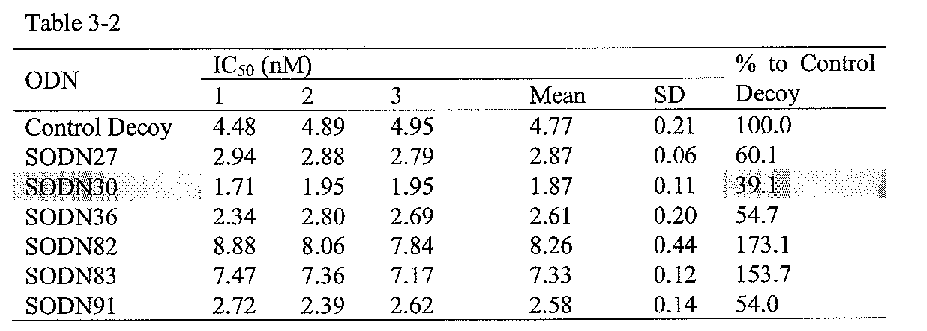

As shown in Tables 3-1 and 3-2, SODNs 7, 8, 9, 16, 17 and 30 (the oligonucleotides of the present invention) showed inhibition activities 2.5 to 3 times higher than that of the control decoy oligonucleotide. The inhibition activity of SODN7 which showed the highest inhibition activity was 5.2 times higher than that of the SODN82 which showed the lowest inhibition activity.

### 4. Binding Capacity of Partially Phosphorothioated Oligonucleotides

The binding capacities of the partially phosphorothioated oligonucleotides (the bonds between only all of the nucleotides constituting the consensus sequence in both strands are phosphorothioated, hereinafter also referred to as "PSODN") of SODNs 7, 8, 9, 16, 17 and 30 of the present invention to the NF-κB (p65 protein) were tested in the same manner as described above. The results are shown in Tables 4 and 5. Table 5 shows the binding capacities of SODNs and PSODNs in comparison. In these tables, the control decoy is the fully phosphorothioated oligonucleotide having the base sequence shown in SEQ ID NO:103. The oligonucleotides to which the same number was assigned, such as, for example, SODN7 and PSODN7, have the same base sequence which is described above.

**Table 4**

| ODN | IC₅₀ (nM) | | | | | % to Control Decoy |
|---|---|---|---|---|---|---|
| | 1 | 2 | 3 | Mean | SD | |
| Control Decoy | 4.42 | 5.39 | 4.97 | 4.93 | 0.40 | 100.0 |
| PSODN7 | 0.46 | 0.53 | 0.63 | 0.54 | 0.07 | 11.0 |
| PSODN8 | 0.53 | 0.64 | 0.72 | 0.63 | 0.08 | 12.8 |
| PSODN9 | 0.35 | 0.66 | 0.61 | 0.54 | 0.14 | 10.9 |
| SODN16 | 0.38 | 0.49 | 0.37 | 0.41 | 0.05 | 8.4 |
| PSODN17 | 0.44 | 0.47 | 0.53 | 0.48 | 0.03 | 9.8 |
| PSODN30 | 0.41 | 0.50 | 0.53 | 0.48 | 0.05 | 9.7 |

**Table 5**

| ODN No. | IC₅₀ (nM) | | % to Control Decoy | |
|---|---|---|---|---|
| | SODN | PSODN | SODN | PSODN |
| 7 | 1.75 | 0.54 | 33.3 | 10.3 |
| 8 | 2.12 | 0.63 | 40.3 | 12.0 |
| 9 | 1.88 | 0.54 | 35.7 | 10.2 |
| 16 | 1.98 | 0.41 | 37.6 | 7.9 |
| 17 | 1.81 | 0.48 | 34.3 | 9.1 |
| 30 | 1.87 | 0.48 | 39.1 | 9.1 |

As shown in Tables 4 and 5 above, PSODNs showed binding capacities to NF-κB (p65 protein) 3.2 to 4.8 times higher than those of the SODNs having the same base sequence, respectively.

### 5. Binding Inhibition Tests to Other Various NF-κB Family Proteins

Whether or not the decoy oligonucleotides (PSODNs 7, 8, 9, 16, 17 and 30) in which only the core was phosphorothioated also inhibits other NF-κB family proteins (p50, p52 and Rel-B) was studied. The study was carried out in basically the same manner as in the above-described primary screening. The activities to inhibit the binding of various NF-κB family proteins to the consensus NF-κB binding site were compared under the conditions of common ratio of 3-fold, 6 times, n=2. As a control for comparison, fully phosphorothioated decoy oligonucleotide ccttgaagggatttccctcc (SEQ ID NO:103) was used. The binding inhibition tests against various NF-κB family proteins were conducted using NF-κB Family TransAM Kit (ACTIVE MOTIF), and using NF-κB molecules in the Jurkat, TPA and CI-Stimulated, Nuclear Extract (ACTIVE MOTIF) for p50, and using NF-κB molecules in the Raji nuclear extract (ACTIVE MOTIF) for Rel-B and p52. As the primary antibodies, anti-NF-κB p50 antibody, anti-NF-κB p52 antibody and anti-Rel-B antibody were used, respectively, and the secondary antibody was HRP-labeled anti-rabbit IgG antibody in all cases.

More concretely, the above-described measurements were carried out as follows: Each oligonucleotide solution was serially diluted with Complete Binding Buffer to prepare test samples. Each of the test samples was added to the wells in an amount of 30 µL per well, and Complete Binding Buffer was added to the wells of the control and blank. Nuclear extract diluted with Complete Lysis Buffer was added to the wells in an amount of 20 µL per well, and Complete Lysis Buffer was added to the wells of blank. After incubation for 1 hour with shaking, each well was washed with 1X Wash Buffer, and the primary antibody was added, followed by incubation for 1 hour. Each well was washed with 1X Wash Buffer, and the secondary antibody was added, followed by incubation for 1 hour. Each well was washed with 1X Wash Buffer, and the Developing Solution was added. After allowing coloration for 10 minutes, the Stop Solution was added to stop the reaction, and the absorbances at 450 nm and 630 nm were measured.

The absorbance at 630 nm was subtracted from the absorbance at 450 nm and the mean value of the blanks was further subtracted from the resultant, and the percentage of the mean value of each concentration based on the mean value of the controls was calculated. The concentration at which the calculated value was 50% was calculated and 50% inhibition concentration (IC₅₀) was calculated using an analysis software Graph Pad PRISM 4, GraphPad SOFTWARE. The results are shown in Table 6. The values for p65 are those obtained in the secondary screening (above-described Table 4).

**Table 6**

| | IC₅₀ (nM) | | | |
|---|---|---|---|---|
| | Rel-B | p52 | p50 | p65* |
| Conventional Type NF-κB Decoy | 9.45 | 8.42 | 8.45 | 4.93 |
| PSODN7 | 1.81 | 3.01 | 1.02 | 0.54 |
| PSODN8 | 1.82 | 3.22 | 1.08 | 0.63 |
| PSODN9 | 1.95 | 1.55 | 0.91 | 0.54 |
| PSODN16 | 2.26 | 2.37 | 0.80 | 0.41 |
| PSODN17 | 1.87 | 4.29 | 0.91 | 0.48 |
| PSODN30 | 0.92 | 2.11 | 0.69 | 0.48 |

Comparing the IC₅₀ values with that of the conventional type NF-κB decoy (fully phosphorothioated SEQ ID NO:103, also same hereinafter), 4.2 to 10.3 times higher binding inhibition activities were observed for Rel-B, 5.4 to 2.0 times higher binding inhibition activities were observed for p52 and 12.2 to 7.8 times higher binding inhibition activities were observed for p50. Thus, it was shown that the novel decoy nucleic acid sequences in which only the core was phosphorothioated have high binding inhibition activities not only for p65 protein but also for other various NF-κB family proteins.

### 5. Binding Inhibition Tests on Decoy Nucleic Acids Having Different Phosphorothioation Sites to NF-κB p65 Protein

To study the influence by the phosphorothioation site in the oligonucleotide sequence, decoy oligonucleotides (FSODN) in which only the flanking sequences were phosphorothioated and decoy oligonucleotides (ODN) without phosphorothioation having the same sequences, respectively, were synthesized. The binding inhibition activities were compared under the conditions of common ratio of 3-fold, 6 times, n=2. The binding inhibition tests against NF-κB p65 protein was carried out using NF-κB, p65 TransAM Kit (produced by ACTIVE MOTIF), and using NF-κB protein molecules in the Jurkat, TPA and CI-Stimulated, Nuclear Extract (produced by ACTIVE MOTIF). The testing method was the same as in the above-described Example 5. The term "only the flanking sequences were phosphorothioated" means that the bonds in the flanking sequences and the bonds between the respective flanking sequences and the core were phosphorothioated. For example, as for Sequence 7, it is CsGsCsGGGATTTCCCsAsGsC. The results of the comparison are shown in Table 7. The values for PSODNs are those obtained in the secondary screening (above-described Table 4).

**Table 7**

| ODN No. ODN No. | IC₅₀ (nM) | | |
|---|---|---|---|
| | PSODN* | FSODN | ODN |
| 7 | 0.54 | >100 | >100 |
| 8 | 0.63 | >100 | >100 |
| 9 | 0.54 | >100 | >100 |
| 16 | 0.41 | 1.11 | >100 |
| 17 | 0.48 | >100 | >100 |
| 30 | 0.48 | >100 | >100 |
| Conventional Type NF-κB Decoy | 4.93 | 4.30 | 3.24 |

Comparing the obtained results with those of the decoy nucleic acids (PSODN) in which only the core sequence was phosphorothioated, the inhibition activities of FSODN and ODN in terms of IC₅₀ were decreased to 100 nM or more. As for FSODN 16, a binding inhibition activity about 3.9 times higher than that of the conventional type NF-κB decoy was observed. Thus, it was suggested that even if the sequence is the same, the binding inhibition activity largely varies depending on the site ofphosphorothioation.

## Claims

1. An oligonucleotide having a base sequence represented by the following Formula [I]:
A-X-B [I]
(wherein in Formula [I], X is a consensus sequence represented by gggatttccc or gggactttcc; A is a 5'-flanking sequence selected from the group consisting of cgc, ccc, gga, cgca, ccct and ggct; and B is a 3'-flanking sequence selected from the group consisting of agc, acc, ggg, gcg, gcc and gcgg).

2. The oligonucleotide according to claim 1, wherein said consensus sequence is gggatttccc.

3. The oligonucleotide according to claim 2, having a base sequence represented by cgcgggatttcccagc, cccgggatttcccacc, ggagggatttcccggg, cgcagggatttcccgcg, ccctgggatttcccgcc or ggctgggatttcccgcgg.

4. The oligonucleotide according to claim 1, wherein said oligonucleotide is double-stranded and the strands are complementary to each other.

5. The oligonucleotide according to claim 1, wherein the bond between at least two adjacent nucleotides is modified by a nuclease-resistant modification.

6. The oligonucleotide according to claim 5, wherein said oligonucleotide is double-stranded, the strands are complementary to each other, and both of the strands are modified by the nuclease-resistant modification.

7. The oligonucleotide according to claim 5, wherein at least the bonds between all of the nucleotides constituting said consensus sequence are modified by the nuclease-resistant modification.

8. The oligonucleotide according to claim 7, wherein the bonds between all nucleotides are modified by the nuclease-resistant modification.

9. The oligonucleotide according to claim 7, wherein the bonds between only all of the nucleotides constituting said consensus sequence are modified by the nuclease-resistant modification, and the bonds between all of other nucleotides are not modified.

10. The oligonucleotide according to claim 5, wherein said nuclease-resistant modification is phosphorothioation.

11. An NF-κB decoy constituted by said oligonucleotide according to claim 1, said oligonucleotide being substantially double-stranded wherein the strands are complementary to each other.

12. The NF-κB decoy according to claim 11, which is completely double-stranded.

13. The NF-κB decoy according to claim 11, which binds to at least one molecular species selected from the group consisting of p65, p50, p52 and Rel-B proteins.

14. A pharmaceutical comprising as an effective ingredient said oligonucleotide according to claim 1, said oligonucleotide being substantially double-stranded wherein the strands are complementary to each other.

15. The pharmaceutical according to claim 14, wherein said oligonucleotide is completely double-stranded.

16. The pharmaceutical according to claim 14, which is an agent for prophylaxis, amelioration and/or therapy of ischemic diseases, allergic diseases, inflammatory diseases, autoimmune diseases, metastasis/infiltration of cancers, or cachexy.

17. The pharmaceutical according to claim 14, which is an agent for prophylaxis, amelioration and/or therapy of vascular restenosis, acute coronary syndrome, brain ischemia, myocardial infarction, reperfusion hindrance of ischemic diseases, atopic dermatitis, psoriasis vulgaris, contact dermatitis, keloid, decubital ulcer, ulcerative colitis, Crohn's disease, nephropathy, glomerulosclerosis, albuminuria, nephritis, renal failure, rheumatoid arthritis, osteoarthritis, degenerative intervertebral disc, asthma, chronic obstructive pulmonary disease (COPD) or cystic fibrosis (CF).

18. The pharmaceutical according to claim 14, which is an agent for prophylaxis, amelioration and/or therapy of vascular restenosis which occurs after percutaneous transluminal coronary angioplasty, percutaneous transluminal angioplasty, bypass surgery, organ transplantation or surgery of an organ.

19. The pharmaceutical according to claim 18, wherein said vascular restenosis is caused by using an artificial blood vessel, catheter or stent, or by vein grafting.

20. The pharmaceutical according to claim 18, wherein said vascular restenosis is caused by a surgical treatment for arteriosclerosis obliterans, aneurysm, aorta dissection, acute coronary syndrome, brain ischemia, Marfan syndrome or plaque rupture.

21. An oligonucleotide decoy for a transcription factor, constituted by an oligonucleotide which is substantially double-stranded wherein the strands are complementary to each other, said oligonucleotide comprising a core sequence and a flanking sequence(s) ligated to one or both ends of said core sequence, **characterized in that** the bonds between only all of the nucleotides constituting said consensus sequence are modified by a nuclease-resistant modification, and the bonds between all of other nucleotides are not modified.

22. The oligonucleotide decoy according to claim 21, wherein said nuclease-resistant modification is phosphorothioation.

23. A method for inhibiting NF-κB, said method comprising bringing said oligonucleotide according to any one of claims 1 to 10 into contact with NF-κB, said oligonucleotide being substantially double-stranded wherein the strands are complementary to each other.

24. Use of said oligonucleotide according to any one of claims 1 to 10 for the production of an inhibitor for inhibiting NF-κB, said oligonucleotide being substantially double-stranded wherein the strands are complementary to each other.

25. A method for prophylaxis, amelioration or therapy of a disease which is cured or ameliorated by inhibition of NF-κB, said method comprising administering an effective amount of said oligonucleotide according to any one of claims 1 to 10, which is substantially double-stranded wherein the strands are complementary to each other.

26. Use of said oligonucleotide according to any one of claims 1 to 10, which is substantially double-stranded wherein the strands are complementary to each other, for the production of a pharmaceutical for a disease which is cured or ameliorated by inhibition of NF-κB.

27. A method for inhibiting a transcription factor, said method comprising making an effective amount of an oligonucleotide decoy for the transcription factor interact with said transcription factor, said oligonucleotide decoy being constituted by an oligonucleotide having a core sequence and a flanking sequence(s) ligated to one or both ends of said core sequence, **characterized in that** the bonds between only all of the nucleotides constituting said consensus sequence are modified by a nuclease-resistant modification, and the bonds between all of other nucleotides are not modified.

28. Use of an oligonucleotide decoy for the production of an inhibitor for inhibiting a transcription factor, said oligonucleotide decoy being constituted by an oligonucleotide having a core sequence and a flanking sequence(s) ligated to one or both ends of said core sequence, **characterized in that** the bonds between only all of the nucleotides constituting said consensus sequence are modified by a nuclease-resistant modification, and the bonds between all of other nucleotides are not modified.
